# EUROPÄISCHE PATENTSCHRIFT

(11) **EP 1 287 794 B1**
(45) Veröffentlichungstag und Bekanntmachung des Hinweises auf die Patenterteilung: **18.06.2008**
(21) Anmeldenummer: 01810827.4
(22) Anmeldetag: 24.08.2001
(51) Int. Cl.: A61F 2/44

(54) **Künstliche Bandscheibe**
ARTIFICIAL SPINAL DISC
DISQUE INTERVERTEBRAL ARTIFICIEL

(43) Veröffentlichungstag der Anmeldung: 05.03.2003
(73) Patentinhaber: Zimmer GmbH, 8404 Winterthur (CH)
(72) Erfinder: Eberlein, Robert, Dr., 8459 Volken (CH); Casutt, Simon, 9200 Gossau (CH); Fröhlich, Markus, 8362 Balterswil (CH)
(74) Vertreter: Manitz, Finsterwald & Partner GbR

(56) Entgegenhaltungen:
- EP-A- 0 346 269
- DE-U- 20 019 520
- FR-A- 2 787 015
- FR-A- 2 787 018
- US-A- 5 674 294
- US-A- 5 683 465

## Beschreibung

Die Erfindung betrifft eine künstliche Bandscheibe gemäss dem Oberbegriff des unabhängigen Anspruchs 1.

Eine derartige künstliche Bandscheibe ist aus FR 2 787 015 A1 bekannt. DE 200 19 520 U1 beschreibt ein Implantat zum Einsetzen in einen Zwischenraum zwischen zwei Wirbelkörpern der Wirbelsäule mit zwei an den zu beiden Seiten des Zwischenraumes angeordneten Wirbelkörpern anlegbaren Stützelementen und mit einem zwischen diesen angeordneten, sich in Längsrichtung des Implantates erstreckenden rohrförmigen Hohlraum, der durch eine balgförmige, in Längsrichtung des Implantates verlängerbare und in Umfangsrichtung des Hohlraumes im wesentlichen nicht dehnbare Außenwand begrenzt wird, wobei die Außenwand von einem Schlauchstück aus einem dicht gewebten oder gewirkten textilen Material gebildet wird.

Die Bandscheibe übernimmt in der Wirbelsäule gleich mehrere zentrale Funktionen. Sie wirkt als Dämpfer, Abstandskörper und auch als Gelenk zwischen den Wirbelkörpern. Entsprechend vielschichtig sind die Anforderungen, die an ein Implantat zu stellen sind, das in seiner Funktion als künstliche Bandscheibe als Ersatz für eine natürliche Bandscheibe dienen soll. So muss die künstliche Bandscheibe selbstverständlich aus biokompatiblen Materialen aufgebaut sein und als Dauerimplantat für den Patienten möglichst lebenslang ihre Funktion erfüllen. Neben der einfachen Funktion als Abstandskörper muss die künstliche Bandscheibe insbesondere die in der Wirbelsäule auftretenden Stosskräfte wirksam abfedern können, damit die Wirbel nicht überlastet werden, ohne dabei jedoch die Wirbelbeweglichkeit merklich zu behindern. Um die natürlichen Dreh-, Kipp- und Schubbelastungen, wie sie in der Wirbelsäule typischerweise auftreten, geeignet ableiten zu können, muss eine feste Verbindung zwischen der künstlichen Bandscheibe und angrenzendem Wirbel gewährleistet sein.

Insbesondere an die elastischen Eigenschaften der künstlichen Bandscheibe, sowohl was deren Verhalten bzgl. Torsions- und Schubbelastungen, als auch in Bezug auf Druckbelastungen angeht, sind somit höchste Anforderungen zu stellen. Insgesamt bedeutet das, dass die mechanischen Eigenschaften der künstlichen Bandscheibe möglichst identisch derjenigen der natürlichen Bandscheibe nachzubilden sind.

Zur Nachahmung der natürlichen Eigenschaften einer Bandscheibe sind zahlreiche Lösungsansätze bekannt. So sind künstliche Bandscheiben bekannt, die aus zwei gegenüber angeordneten Endplatten bestehen, die über ein elastisches Stützelement verbunden sind, das die Funktion eines künstlichen Nucleus übernimmt und mit den Endplatten verklebt ist. Der künstliche Nucleus kann dabei aus einer Kombination von verschiedenen elastischen Kunststoffen bestehen, die noch eine Ummantelung aufweisen können. Das bisher ungelöste Problem solcher Anordnungen besteht jedoch unter anderem darin, die passende, d.h. die natürliche, stark nicht-lineare, Charakteristik einer Bandscheibe im üblichen Belastungsbereich für Druck-, Zug-, Schub-, und Torsionsbeanspruchungen bei einer künstlichen Bandscheibe nachzubilden.

Die Aufgabe der Erfindung ist es daher, eine künstliche Bandscheibe vorzuschlagen, welche die komplexen elastischen Eigenschaften einer natürlichen Bandscheibe möglichst exakt nachbildet.

Die diese Aufgaben lösende künstliche Bandscheibe ist durch die Merkmale des unabhängigen Anspruchs 1 gekennzeichnet. Die abhängigen Ansprüche beziehen sich auf besonders vorteilhafte Ausführungsformen der Erfindung.

Die erfindungsgemässe künstliche Bandscheibe zum Implantieren zwischen zwei Wirbelkörper, umfasst zwei Endplatten und eine elastisch verformbare, zwischen den Endplatten in axialer Richtung unter Vorspannung verspannte Scheibe, wobei die Scheibe innerhalb eines rohrförmigen elastisch verformbaren Faserrings liegt. Dabei stehen die Endplatten mit dem Faserring in zugfester Verbindung. Die Scheibe bildet eine, mit einer Kompression der künstlichen Bandscheibe sich vergrössernde Berührungsfläche mit einer der angrenzenden Endplatten und die elastischen Eigenschaften der künstlichen Bandscheibe zeigen, mindestens in Bezug auf eine Kompressionskraft, mit zunehmender Verformung ein nicht-lineares Verhalten.

Bei der erfindungsgemässen künstlichen Bandscheibe sind zwei sich gegenüberstehende Endplatten an ihrer Peripherie mittels eines elastisch verformbaren Faserrings verbunden, welcher auf die Endplatten im unbelasteten Zustand eine ständige Zugkraft ausübt, so dass die Scheibe, die sich zwischen den beiden Endplatten innerhalb des Faserrings befindet, unter Druckvorspannung steht. Ein wesentlicher Nachteil bekannter künstlicher Bandscheiben besteht darin, dass unter der Wirkung einer elastischen Deformation zuviel Weg beansprucht wird, bis der eigentliche Belastungsbereich erreicht ist. Ein entscheidender Vorteil der erfindungsgemässen künstlichen Bandscheibe ist daher darin zu sehen, dass deren elastisches Verhalten, zumindest in Bezug auf eine Kompression, bereits bei kleinen Deformationen ein nicht-lineares Verhalten zeigt.

Das elastische Verhalten der erfindungsgemässen künstlichen Bandscheibe ist dabei durch die konstruktiven Besonderheiten von Faserring und Scheibe und der sie umfassenden Materialien, sowie durch die konstruktionsbedingte mechanische Kopplung von Faserring und Scheibe bestimmt. So kann das mechanische Verhalten des Faserrings bezüglich einer Dehnung unter Zugbeanspruchung, sowie einer Dehnung z.B. infolge von Torsionsbelastungen schon durch die Art des Gewebes verschieden sein, je nachdem ob der Faserring gewebt, gestrickt, geflochten oder auf andere Weise hergestellt wurde. Dabei spielt unter anderem die Orientierung der Fasern, aus denen der Faserring aufgebaut ist, in ihrer Schräglage zur Richtung der Längsachse der künstlichen Bandscheibe eine Rolle für die zwischen den Endplatten zu übertragenden Kräften. Während die Scheibe alleine, d.h. nicht in Kombination mit dem Faserring, z.B. in Bezug auf reine Torsionsbewegungen lediglich einen Gleitreibungswiderstand bildet, kann durch die Kombination beider Elemente und eine entgegengesetzte Schrägstellung der Fasern relativ zur Richtung der Längsachse der künstlichen Bandscheibe, eine Umsetzung z.B. von Torsionsbewegungen in eine Kompression der Scheibe erreicht werden. Dadurch werden die nicht-linearen elastischen Eigenschaften, die die Scheibe bezüglich einer Kompression aufweist, in das elastische Torsionsverhalten der künstlichen Bandscheibe transformiert. Die Scheibe steht im unbelasteten Zustand über den Faserring unter einer gewissen Druckvorspannung. Damit besteht die Möglichkeit, unter Einhaltung einer vorgegebenen Geometrie für die künstlichen Bandscheibe und ohne Veränderung oder Austausch der die künstliche Bandscheibe aufbauenden Materialien, die elastischen Eigenschaften an die individuellen Bedürfnisse eines Patienten anzupassen. So kann die nicht-lineare Belastungscharakteristik z.B. an das Körpergewicht des Patienten und / oder die Kopplung von Torsionsbelastungen oder anderen Belastungsarten an eine Kompression der Scheibe angepasst werden.

Die Art der Konstruktion mit einem auf Zug beanspruchten Faserring und einer auf Druck beanspruchten Scheibe gestattet es, die Vorspannung zwischen diesen beiden Elementen im unbelasteten Zustand so hoch zu wählen, dass bei extremer Druckbelastung immer noch eine Restspannung in Zugrichtung vorhanden ist, die bei einer Begrenzung von zusätzlichen Scher- und Torsionsbelastungen mitwirkt. Darüber hinaus kann man (z.B. durch die Geometrie der Scheibe in Kombination mit einer geeigneten Wahl der Vorspannung des Faserrings) es erreichen, dass die künstliche Bandscheibe für kleine Belastungen bezüglich Kipp- und Biegebewegungen eine gewisse elastische Neutralzone aufweist.

Die den Faserring aufbauenden Materialien umfassen, soweit sie mit Körpergewebe in Berührung kommen, körperverträgliche Kunststoffe. Der Faserring selbst kann durchlässig für körpereigene Flüssigkeiten sein. Die Endplatten können aus einem Metall, beispielsweise aus zähfestem Titan oder aus einer zähfesten Titanlegierung gefertigt sein, welche für die Verankerung des Faserrings plastisch deformierbar ist. Die Aussenflächen der Endplatten können Zonen mit Metallgewebe aufweisen, die das Einwachsen von Knochenmaterial, und damit das Verwachsen der angrenzenden Wirbel mit den äusseren Flächen der Endplatten erleichtern. Eine Verbesserung der Verankerung zwischen Endplatte und Wirbel bezüglich Seitenkräften wird durch eine oder mehrere vorstehende Rippen erreicht, die beispielsweise von ventral nach dorsal verlaufen können. Eine zusätzliche Zahnung am Aussenrand der Endplatte, die, ebenso wie die vorstehende Rippe, nicht zwingend vorhanden sein muss, kann ebenfalls die Verbindung zwischen Endplatte und Wirbel bezüglich Scherbeanspruchungen deutlich verbessern. Dabei muss die Endplatte nicht unbedingt aus Metall aufgebaut sein, sondern es können beispielsweise auch geeignete körperverträgliche Kunststoffe als Materialien für den Aufbau der Endplatte in Betracht kommen.

Im folgenden wird die Erfindung anhand von bevorzugten Ausführungsbeispielen, sowie der Zeichnung näher erläutert. Es zeigen:
- Fig. 1: Schematisch einen Längsschnitt durch eine erste Variante einer erfindungsgemässen künstliche Bandscheibe;
- Fig. 2: eine zweite Variante des Ausführungsbeispiels gemäss Fig. 1;
- Fig. 3: eine dritte Variante des Ausführungsbeispiels gemäss Fig. 1
- Fig. 4: eine vierte Variante des Ausführungsbeispiels gemäss Fig. 1
- Fig. 5: eine andere Variante des Ausführungsbeispiels gemäss Fig. 1
- Fig. 6: schematisch eine Ansicht gemäss den Figuren 1 bis 5;
- Fig. 7: eine erfindungsgemässe künstliche Bandscheibe gemäss den Figuren 1 bis 6, eingesetzt zwischen zwei Wirbel;
- Fig. 8: Kraft - Weg Diagramm des elastischen Verhaltens einer erfindungsgemässen künstlichen Bandscheibe;
- Fig. 9: ein Ausführungsbeispiel zur Verbindung des Faserrings mit einer Endplatte mittels eines ringförmigen Klemmelementes;
- Fig. 10: eine zweite Variante eines Ausführungsbeispiels gemäss Fig. 9;
- Fig. 11: eine dritte Variante eines Ausführungsbeispiels gemäss Fig. 9, wobei der Faserring zusätzlich verklebt oder verschweisst ist;
- Fig. 12: ein Ausführungsbeispiel zur Verbindung des Faserrings mit einer Endplatte, wobei der Faserring verklebt, verschweisst oder angespritzt ist;
- Fig. 13: ein Ausführungsbeispiel zur Verbindung des Faserrings mit einer Endplatte, wobei der Faserring durch ein Halteelement fixiert ist;
- Fig. 14: ein weiteres Ausführungsbeispiel gemäss Fig. 13;
- Fig. 15: ein Ausführungsbeispiel zur Verbindung des Faserrings mit einer Endplatte, wobei der Faserring in einer Nut kalt verpresst ist;
- Fig. 16: ein zweites Ausführungsbeispiel gemäss Fig. 15;
- Fig. 17: ein drittes Ausführungsbeispiel gemäss Fig. 15.

Die Erfindung wird im folgenden anhand bevorzugter Ausführungsbeispiele erläutert. Die erfindungsgemässe künstliche Bandscheibe B umfasst zwei Endplatten 1, 2, zwischen welchen mittels eines Faserrings 4 eine Scheibe 3 unter Vorspannung in axialer Richtung verspannt ist, wodurch die Endplatten 1, 2 in zugfester Verbindung stehen. Dabei weist die Scheibe 3 unter Druckbelastung eine sich vergrössernde Berührungsfläche 5, 6 mit einer der angrenzenden Endplatten 1, 2 auf. Die elastischen Eigenschaften der künstlichen Bandscheibe B zeigen dabei mindestens in Bezug auf eine Kompressionskraft F ein mit zunehmender Deformation S nicht lineares Verhalten.

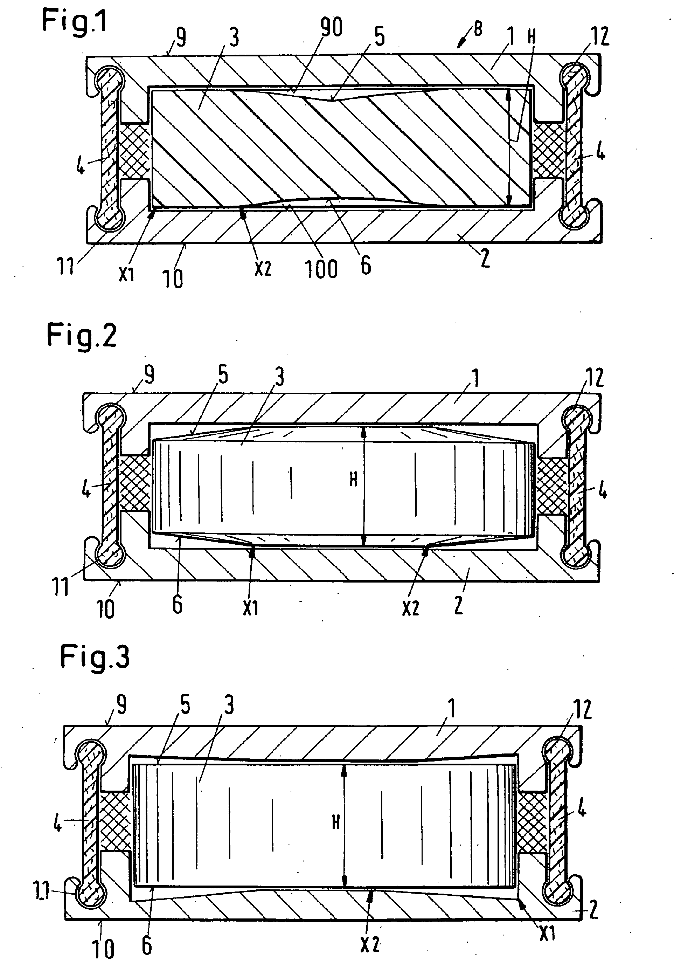

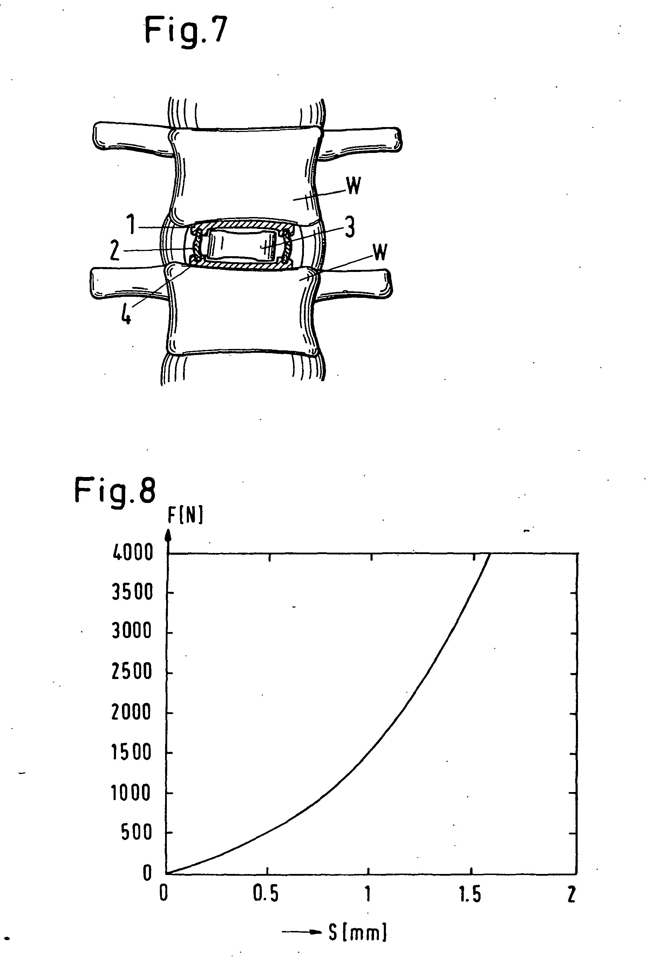

Fig. 1 zeigt in einer schematischen Darstellung ein bevorzugtes Ausführungsbeispiel einer erfindungsgemässen künstlichen Bandscheibe B. Zwischen den Endplatten 1, 2, deren Innenseiten 90, 100 als plane Flächen ausgebildet sind, ist eine Scheibe 3 in radialer Richtung unter Vorspannung verspannt. Die Höhe H der Scheibe 3 des bevorzugten Ausführungsbeispiels aus Fig. 1 verringert sich in radialer Richtung von aussen nach innen, wobei jedoch die Höhe H auch im unbelasteten Zustand am Rand zwischen X1, X2 zunächst konstant bleibt. Der Abstand von X1 zu X2 hängt von der Grösse des Schubmoduls der Scheibe in der Nähe von X1 ab. Der Schubmodul der Scheibe 3 nimmt dabei in radialer Richtung von aussen nach innen zu. Unter einer Kompressionskraft F, die senkrecht zu den Flächen 1, 2 auf die künstliche Bandscheibe B wirkt, wird die elastische Scheibe 3 komprimiert, wobei sich die Berührungsfläche 5, 6 zwischen Scheibe 3 und Innenfläche 90, 100 der Deckplatte 1, 2 vergrössert. Da der Schubmodul der Scheibe 3 von aussen nach innen in radialer Richtung anwächst, erhöht sich mit zunehmender Kompression insgesamt die Steifigkeit der künstlichen Bandscheibe B. Dieses Verhalten zeigt Fig. 8, in der exemplarisch in Form eines Kraft-Weg Diagramms die Deformation S einer erfindungsgemässen künstlichen Bandscheibe B unter einer Kompressionskraft F aufgetragen ist. Wobei eine grössere Steifigkeit bedeutet, dass für eine bestimmte fest vorgegebene Deformation S der künstlichen Bandscheibe B eine grössere Kraft F benötigt wird, als für eine gleiche Deformation S bei einer kleineren Steifigkeit. Das heisst, die Steifigkeit korrespondiert mit der Steigung der Kurve aus Fig. 8. Eine höhere Steifigkeit entspricht einem steileren Anstieg der Kurve. So liegt beispielsweise die Steifigkeit der erfindungsgemässen künstlichen Bandscheibe unter einer Kompressionskraft F von ca. 2500 N in einem Bereich zwischen 1700 N/mm und 4500 N/mm. Es ist ein besonderes Charaktersitikum des Kraft-Weg Diagramms in Fig. 8, dass, analog zum Verhalten einer natürlichen Bandscheibe, die Anfangssteigung der Kurve einen positiven, von Null verschiedenen Wert hat. Das heisst, die erfindungsgemässe künstliche Bandscheibe B zeigt bereits bei beliebig kleiner Kompressionskraft F und damit bei beliebig kleinen Belastungen, eine gewisse Anfangssteifigkeit, die von Null verschieden ist. Dabei kann die Bauhöhe der künstlichen Bandscheibe B entweder konstant sein oder z.B. von anterior nach posterior abnehmen. Das kann beispielweise dadurch realisiert werden, dass die Höhe H der Scheibe 3 z.B. von anterior nach posterior abnimmt oder die Dicke der Endplatten 1, 2 entsprechend variiert. Dadurch kann unter anderem erreicht werden, dass das elastische Verhalten der künstlichen Bandscheibe B bezüglich Flexions- und Extensionsbewegungen nicht symmetrisch ist.

Da die nicht-linearen Eigenschaften der Scheibe 3, die diese bezüglich einer Kompressionskraft F aufweist, durch den Faserring 4 in das elastische Torsions- und Flexionsverhalten transformiert werden, zeigt die künstliche Bandscheibe B auch bei entsprechenden beliebig kleinen Auslenkungen aus dem unbelasteten Zustand, gegenüber Torsions- und Flexionsbewegungen eine bestimmte Anfangssteifigkeit. Da die erwähnte Anfangssteifigkeit dadurch erreicht wird, dass die Scheibe 3 im unbelasteten Zustand über den Faserring 4 unter einer gewissen Druckvorspannung steht, ergibt sich die Möglichkeit, unter Einhaltung einer vorgegebenen Geometrie für die künstlichen Bandscheibe B und ohne Veränderung oder Austausch der die künstliche Bandscheibe B aufbauenden Materialien, die elastischen Eigenschaften an die individuellen Bedürfnisse eines Patienten anzupassen. So kann die nicht-lineare Belastungscharakteristik und damit die Steifigkeit und deren Veränderung mit zunehmender Kompressionskraft F z.B. an das Körpergewicht des Patienten angepasst werden, indem einfach durch eine geeignete Wahl der Druckvorspannung entsprechend den Anforderungen, die durch den Körperbau des Patienten individuell definiert werden, angepasst werden.

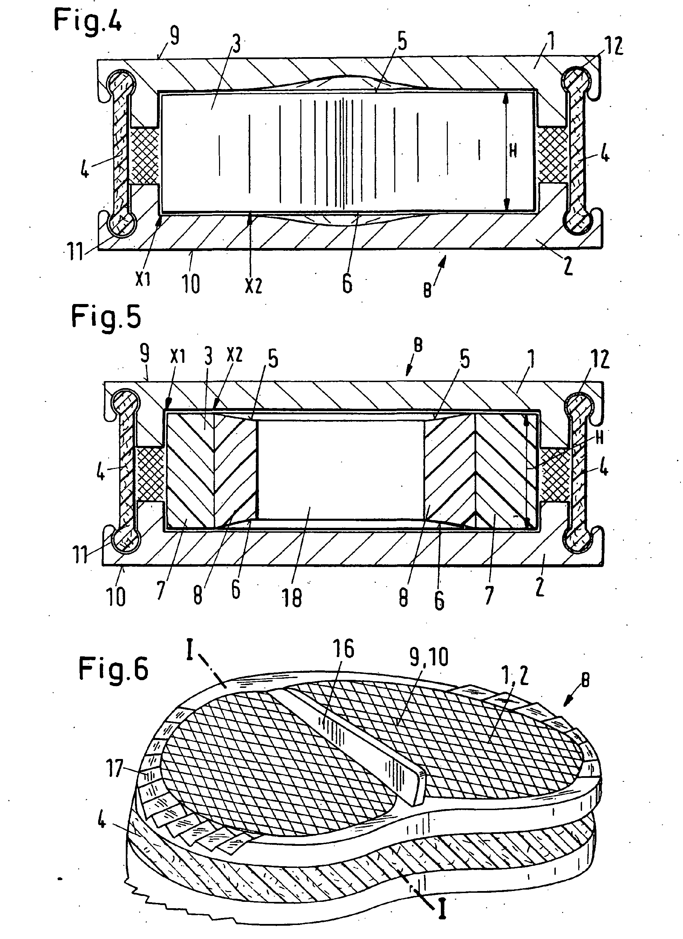

Die nicht-linearen Eigenschaften der Scheibe 3 bezüglich einer Kompressionskraft F werden im wesentlichen durch die Kombination zweier Merkmale erreicht. Erstens vergrössert sich mit zunehmendem Kompressionsweg S die Berührungsfläche 90, 100 zwischen der Scheibe 3 und der Endplatte 1, 2. Zweitens nimmt der Schubmodul der Scheibe 3 in radialer Richtung in der Weise zu, dass die Steifigkeit der künstlichen Bandscheibe mit zunehmender Kompression grösser wird. Somit kann die Charakteristik des elastischen Verhaltens, wie es in Figur 8 exemplarisch gezeigt wird, auch über die Geometrie der Scheibe 3, z.B. durch Variation der Breite des Bereiches zwischen X1 und X2, festgelegt werden. Auch durch eine geeignete Wahl der Materialien, aus denen die Scheibe aufgebaut wird, lassen sich die nicht-linearen Eigenschaften der Scheibe 3 einstellen. Darüber hinaus kann die Stärke der Zunahme des Schubmoduls der Scheibe 3 in radialer Richtung auf verschiedene Weisen festgelegt bzw. variiert werden. Die Scheibe 3 kann zum Beispiel aus Silikonelastomeren oder Gummimaterialien wie z.B. Polycarbourethan (PCU) aufgebaut sein. Solche Materialen können zur Herstellung der Scheibe 3 angespritzt und in den verschiedenen Bereichen der Scheibe 3 unterschiedlich stark nachverpresst werden, wodurch in der Scheibe 3 Bereiche unterschiedlicher Steifigkeit erzeugt werden können. Eine andere Möglichkeit das nicht-lineare Verhalten der Scheibe 3 zu modellieren besteht darin, die Scheibe 3 konzentrisch aus wirkfest miteinander verbundenen Ringen verschiedener Materialien, die unterschiedliche Steifigkeit aufweisen, aufzubauen. Dabei kann die Scheibe 3 wie in Fig. 5 gezeigt so aus wirkfest miteinander verbundenen Ringteilen 7, 8 aufgebaut sein, dass die Ringeteile 7, 8 einen Hohlraum 18 umschliessen. Die Bildung des Hohlraumes 18 ist jedoch in keiner Weise zwingend erforderlich, d.h. der Hohlraum 18 kann auch fehlen.

Für die Bildung der sich unter einer Kompressionskraft F vergrössernde Fläche 5, 6 sind verschiedene Varianten denkbar. So kann die nach innen gewandte Fläche 90, 100 der Endplatte 1, 2 wie in den Fig. 1, 2, 5 gezeigt, eine im wesentlichen plane Oberfläche aufzeigen. Die Höhe H der Scheibe 3 verringert sich dann in radialer Richtung entweder von aussen nach innen (siehe Fig. 1 und Fig 5) oder von innen nach aussen (siehe Fig 2), wobei die Höhe H auch im unbelasteten Zustand über den Bereich zwischen X1 und X2 konstant bleibt. Weitere Varianten werden in den Fig. 3 und 4 gezeigt. Bei diesen Varianten des bevorzugten Ausführungsbeispiels ist die Höhe H der Scheibe 3 konstant. Stattdessen variiert der Abstand der Deckplatte 1, 2 zur Scheibe 3 in radialer Richtung entweder von innen nach aussen oder umgekehrt. Dabei zeigen die in den Fig. 1, 4, 5 gezeigten Varianten eine besonders hohe Stabilität in Bezug auf Kippbelastungen, während die Varianten, die in den Fig. 2 und 3 gezeigt werden, eine grössere Biegeelastizität haben können. Selbstverständlich sind auch geeignete Kombinationen der zuvor beschriebenen Ausführungsbeispiele denkbar.

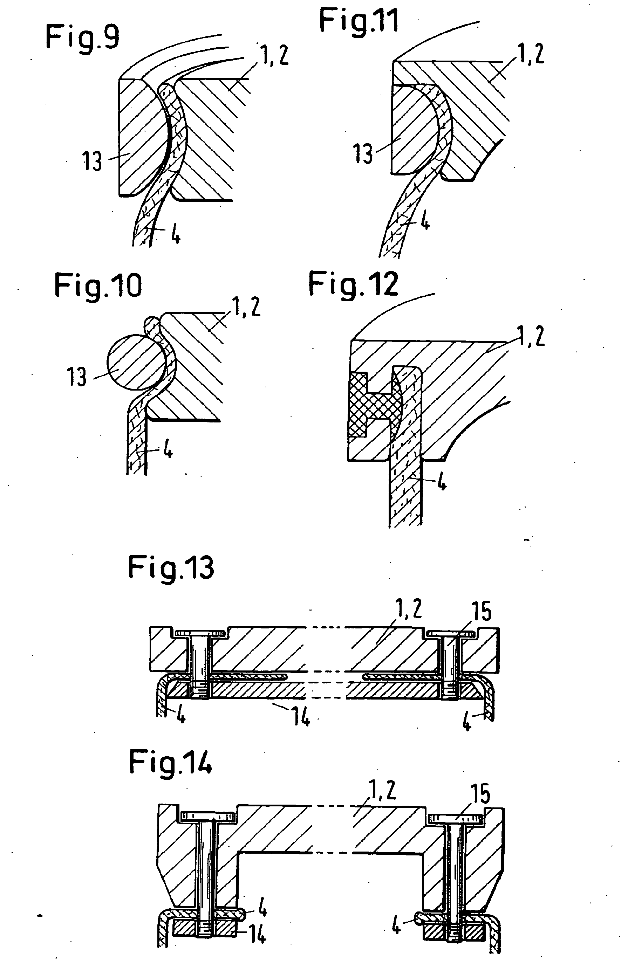

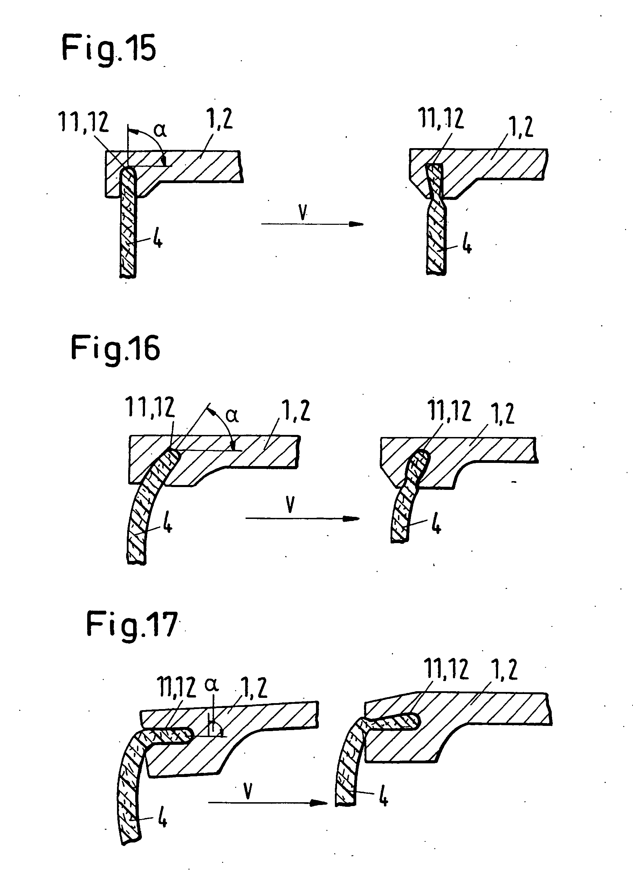

Voraussetzung für eine gute Funktion der erfindungsgemässen künstlichen Bandscheibe ist eine einwandfreie Verankerung des Faserrings 4 in der Endplatte 1, 2. In den Fig. 9 bis 12 und den Fig. 15 bis 17 ist schematisch eine Nut 11, 12 in der Endplatte 1, 2 gezeigt, in der der Faserring 4 befestigt ist. Die Art der Befestigung hängt unter anderem vom Material der Endplatte 1, 2 ab. So kann der Faserring 4, wie in den Fig. 9, 10 gezeigt, an der Endplatte 1, 2, die aus Metall oder Kunststoff gefertigt ist, z.B. durch ein ringförmiges Klemmelement 13 in der Nut 11, 12 fixiert werden und / oder zusätzlich (siehe Fig. 11, 12) in der Nut 11, 12 verklebt sein. Bei der Endplatte 1, 2 aus Kunststoff ist alternativ zum Kleben auch thermisches Verschweissen in der Nut 11, 12 oder auf einer Oberfläche der Endplatte 1, 2 möglich. Bei einer metallischen Endplatte 1, 2 kann der Faserring 4 durch Kaltumformung V in der Nut 11, 12 verpresst werden. Dabei kann die Nut 11, 12 wie in den Fig. 15 bis 17 gezeigt unter einem bestimmten Winkel α gegen die Oberfläche der Endplatte 1, 2 geneigt sein. Dabei wird ein Winkel α gewählt, der zwischen 0 Grad (Fig. 17) und 90 Grad (Fig. 15), vorzugsweise jedoch in der Nähe von 30 Grad liegt. Als weitere Variante für die Befestigung des Faserrings 4 an der Endplatte 1, 2 ist auch eine Fixierung mittels eines zusätzlichen Halteelementes 14 denkbar, das zum Beispiel als Platte (Fig. 13) oder als Ring (Fig. 14) ausgebildet sein kann und vorzugsweise mittels einer Verschraubung 15 an der Endplatte 1, 2 befestigt ist.

Ein weiterer wesentlicher Punkt ist die Verankerung der künstlichen Bandscheibe B in der menschlichen Wirbelsäule an einem angrenzenden Wirbel W (siehe Fig. 7). So kann eine Verbesserung der Verankerung zwischen Endplatte 1, 2 und angrenzendem Wirbel W bezüglich Seitenkräften durch eine oder mehrere vorstehende Rippen 16, die z.B. von ventral nach dorsal verlaufen, wesentlich verbessert werden. Eine zusätzliche Zahnung 17 am Aussenrand der Deckplatte 1, 2 verbessert ebenfalls die Verbindung zwischen Deckel und Wirbel, insbesondere in Bezug auf Scherbeanspruchungen. Auf den Aussenseiten der Endplatten 1, 2 können Zonen aus Metallgeflechten, vorzugsweise aus Titan oder Titanlegierungen, aufgebracht sein, die das Einwachsen von Knochenmaterial an den Wirbeln erleichtern bzw. befördern.

## Patentansprüche

1. Künstliche Bandscheibe, zum Implantieren zwischen zwei Wirbelkörper (W), umfassend zwei Endplatten (1, 2) und eine elastisch verformbare, zwischen den Endplatten (1, 2) in axialer Richtung unter einer Vorspannung verspannte Scheibe (3), wobei die Scheibe (3) eine, mit einer Kompression der künstlichen Bandscheibe sich vergrössernde Berührungsfläche (5, 6) mit einer der angrenzenden Endplatten (1, 2) aufweist, und die elastischen Eigenschaften der künstlichen Bandscheibe mindestens in Bezug auf eine Kompressionskraft (F) mit zunehmender Verformung ein nicht-lineares Verhalten zeigen,
**dadurch gekennzeichnet, dass**
die Scheibe (3) innerhalb eines rohrförmigen elastisch verformbaren Faserrings (4) liegt und die Endplatten (1, 2) mit dem Faserring (4) in zugfester Verbindung stehen.

2. Künstliche Bandscheibe nach Anspruch 1, bei welcher der Wert für die Steifigkeit der künstlichen Bandscheibe unter einer Kompressionskraft (F) von 2500 N in einem Bereich zwischen 1700 N/mm und 4.500 N/mm liegt.

3. Künstliche Bandscheibe nach Anspruch 1 oder 2, bei welcher der Schubmodul der Scheibe (3) in radialer Richtung derart zunimmt, dass unter einer zunehmenden Kompressionskraft (F) die Steifigkeit der künstlichen Bandscheibe ebenfalls zunimmt.

4. Künstliche Bandscheibe nach Anspruch 1 bis 3, bei welcher der Faserring (4) aus einem Gewebe, Gewirk oder Geflecht besteht und eine oder mehrere Lagen umfasst.

5. Künstliche Bandscheibe nach einem der Ansprüche 1 bis 4, bei welcher die den Faserring (4) aufbauenden Materialien einen oder mehrere Kunststoffe, wie z.B. Polyamid, Polyimid, Kohlefasern oder Polyetheretherketon (PEEK), vorzugsweise Polyethylen Terephthalat (PET), umfassen.

6. Künstliche Bandscheibe nach einem der Ansprüche 1 bis 5, bei welcher die Scheibe (3) zwei konzentrisch angeordnete, wirkfest miteinander verbundene Ringteile (7, 8) umfasst.

7. Künstliche Bandscheibe nach einem der Ansprüche 1 bis 6, bei welcher die die Scheibe (3) aufbauenden Materialien Silikonelastomere oder Gummimaterialien, wie z.B. Polycarbourethan (PCU), umfassen.

8. Künstliche Bandscheibe nach einem der Ansprüche 1 bis 7, bei welcher die Endplatten (1, 2) aus einem Metall, vorzugsweise aus Titan, einer Titanlegierung oder aus Kunststoff bestehen.

9. Künstliche Bandscheibe nach einem der Ansprüche 1 bis 8, bei welcher die Endplatten (1, 2) als Verankerungsplattformen ausgebildet sind, um ein Verwachsen der angrenzenden Wirbelkörper (W) mit den nach aussen gerichteten Flächen (9, 10) der Endplatten (1, 2) zu erreichen.

10. Künstliche Bandscheibe nach einem der Ansprüche 1 bis 9, an welcher eine der Endplatten (1, 2) eine in Umfangsrichtung verlaufende Nut (11, 12) zur Aufnahme des Faserrings (4) aufweist.

11. Künstliche Bandscheibe nach Anspruch 10, an welcher der Faserring (4) zur Verbindung mit einer der Endplatten (1, 2) durch Kaltumformung (V) in einer der Nuten (11, 12) verpresst ist.

12. Künstliche Bandscheibe nach Anspruch 10 oder 11, an welcher der Faserring (4) zur Verbindung mit einer der Endplatten (1, 2) durch ein ringförmiges Klemmelement (13) in einer der Nuten (11, 12) fixiert ist.

13. Künstliche Bandscheibe nach einem der Ansprüche 1 bis 12, an welcher der Faserring (4) zur Verbindung mit einer der Endplatten (1, 2) durch ein Halteelement (14), vorzugsweise mittels einer Verschraubung (15) fixiert ist.

14. Künstliche Bandscheibe nach einer der Ansprüche 10 bis 13, an welcher der Faserring (4) zur Verbindung mit einer der Endplatten (1, 2) in einer der Nuten (11, 12) verklebt oder verschweisst ist.

15. Künstliche Bandscheibe nach einem der Ansprüche 10 bis 14, an welcher der Faserring (4) zur Verbindung mit einer der Endplatten (1, 2) ringförmig in einer der Nuten (11, 12) der Endplatten (1, 2) mit Kunststoff angespritzt ist.

16. Künstliche Bandscheibe nach einem der Ansprüche 1 bis 15, an welcher der Faserring (4) zur Verbindung mit einer der Endplatten (1, 2) auf einer der Oberflächen (9, 10) derselben mit Kunststoff angespritzt ist.

17. Künstliche Bandscheibe nach einem der Ansprüche 1 bis 16, bei welcher die Endplatten (1,2) eine vorstehende mittlere Rippe (16) aufweisen, die beispielsweise von ventral nach dorsal verläuft.

18. Künstliche Bandscheibe nach einem der Ansprüche 1 bis 17, bei welcher die Endplatten (1,2) an ihrem Aussenrand eine zum Wirbel (W) hin gerichtete Zahnung (17) aufweisen, so dass durch ein Einwachsen von Knochenmaterial eine formschlüssige Verbindung für die Übertragung von Torsions- Biege- und Schubkräften erreicht wird.

19. Künstliche Bandscheibe nach einem der Ansprüche 1 bis 18, bei welcher im unbelasteten Zustand die Vorspannung, unter die die Scheibe (3) durch den Faserring (4) gesetzt wird, so hoch ist, dass bei einer maximalen Druckbelastung von beispielsweise 4000 N der Faserring (4) immer noch eine positive Restspannung in Zugrichtung aufweist.

## Claims

1. An artificial intervertebral disc for implantation between two vertebral bodies (W) comprising two end plates (1, 2) and an elastically deformable disc (3) stressed under pre-stress between the end plates (1, 2) in the axial direction, wherein the disc (3) has a contact surface (5, 6) with one of the adjacent end plates (1, 2) which enlarges with a compression of the artificial intervertebral disc and the elastic properties of the artificial intervertebral disc show a nonlinear behaviour with increasing deformation at least with respect to a compression force (F),
**characterised in that**
the disc (3) is disposed within a tubular, elastically deformable, fibre ring (4) and the end plates (1, 2) are in connection resistant to tension with the fibre ring (4),.

2. An artificial intervertebral disc in accordance with claim 1, wherein the value for the stiffness of the artificial intervertebral disc under a compression force (F) of 2,500 N lies in a range between 1,700 N/mm and 4,500 N/mm.

3. An artificial intervertebral disc in accordance with claim 1 or claim 2, wherein the shear modulus of the disc (3) increases in the radial direction such that the stiffness of the artificial intervertebral disc likewise increases with an increasing compression force (F).

4. An artificial intervertebral disc in accordance with any one of the claims 1 to 3, wherein the fibre ring (4) consists of a fabric, a knitted fabric or a braid and comprises one or more layers.

5. An artificial intervertebral disc in accordance with any one of the claims 1 to 4, wherein the materials building up the fibre ring (4) comprise one or more plastics such as polyamide, polyimide, carbon fibres or polyether etherketone (PEEK), preferably polyethylene terephthalate (PET).

6. An artificial intervertebral disc in accordance with any one of the claims 1 to 5, wherein the disc (3) comprises two concentrically arranged ring parts (7, 8) mutually effectively fixedly connected.

7. An artificial intervertebral disc in accordance with any one of the claims 1 to 6, wherein the materials building up the disc (3) comprise silicon elastomers or rubber materials such as polycarbourethane (PCU).

8. An artificial intervertebral disc in accordance with any one of the claims 1 to 7, wherein the end plates (1, 2) consist of a metal, preferably of titanium, of a titanium alloy or of plastic.

9. An artificial intervertebral disc in accordance with any one of the claims 1 to 8, wherein the end plates (1, 2) are formed as anchoring platforms in order to achieve a growing together of the adjacent vertebral bodies (W) with the outwardly directed areas (9, 10) of the end plates (1, 2).

10. An artificial intervertebral disc in accordance with any one of the claims 1 to 9, at which one of the end plates (1, 2) has a groove (11, 12) extending in the peripheral direction in order to receive the fibre ring (4).

11. An artificial intervertebral disc in accordance with claim 10, at which the fibre ring (4) is pressed in one of the grooves (11, 12) by cold forming (V) for the connection to one of the end plates (1, 2).

12. An artificial intervertebral disc in accordance with claim 10 or claim 11, at which the fibre ring (4) is fixed in one of the grooves (11, 12) by an annular clamping element (13) for the connection to one of the end plates (1, 2).

13. An artificial intervertebral disc in accordance with any one of the claims 1 to 12, at which the fibre ring (4) is fixed by a holding element (14), preferably by means of a screw connection (15), for the connection to one of the end plates (1, 2).

14. An artificial intervertebral disc in accordance with any one of the claims 10 to 13, at which the fibre ring (4) is adhesively bonded or welded in one of the grooves (11, 12) for the connection to one of the end plates (1, 2).

15. An artificial intervertebral disc in accordance with any one of the claims 10 to 14, at which the fibre ring (4) is injection moulded with plastic in an annular manner in one of the grooves (11, 12) of the end plates (1, 2) for the connection to one of the end plates (1, 2).

16. An artificial intervertebral disc in accordance with any one of the claims 1 to 15, at which the fibre ring (4) is injection moulded with plastic for the connection to one of the end plates (1, 2) at one of the surfaces (9, 10) thereof.

17. An artificial intervertebral disc in accordance with any one of the claims 1 to 16, wherein the end plates (1, 2) have a projecting central rib (16), which extends, for example, from ventral to dorsal.

18. An artificial intervertebral disc in accordance with any one of the claims 1 to 17, wherein the end plates (1, 2) have a toothed arrangement (17) directed to the vertebra (W) at their outer rim such that a shape-locked connection is achieved by an ingrowth of bone material for the transmission of torsional, bending and shear forces.

19. An artificial intervertebral disc in accordance with any one of the claims 1 to 18, wherein the pre-stress under which the disc (3) is put by the fibre ring (4) in the unloaded state is so high that with a maximum pressure stress of, for example, 4,000 N, the fibre ring (4) still always has a positive residual stress in the tension direction.

## Revendications

1. Disque intervertébral artificiel, destiné à être implanté entre deux vertèbres (W), comprenant deux plaques terminales (1, 2) et un disque élastiquement déformable (3) mis sous précontrainte en direction axiale entre les plaques terminales (1, 2), dans lequel le disque (3) présente une surface de contact (5, 6), qui s'agrandit avec une compression du disque intervertébral artificiel, avec l'une des plaques terminales (1, 2) adjacentes, et les propriétés élastiques du disque intervertébral artificiel présentent un comportement non linéaire au fur et à mesure que la déformation augmente, au moins par référence à une force de compression (F),
**caractérisé en ce que**
le disque (3) est placé à l'intérieur d'une bague fibreuse (4) de forme tubulaire et élastiquement déformable, et les plaques terminales (1, 2) se trouvent dans une liaison résistante à la traction avec la bague fibreuse (4).

2. Disque intervertébral artificiel selon la revendication 1, dans lequel la valeur pour la raideur du disque intervertébral artificiel sous une force de compression (F) de 2500 N est située dans une plage entre 1700 N/mm et 4500 N/mm.

3. Disque intervertébral artificiel selon la revendication 1 ou 2, dans lequel le module d'élasticité transversale du disque (3) en direction radiale augmente de telle façon que sous une force de compression croissante (F) la raideur du disque intervertébral artificiel augmente également.

4. Disque intervertébral artificiel selon l'une des revendications 1 à 3, dans lequel la bague fibreuse (4) est réalisée en tissu, en textile ou en treillis et comprend une ou plusieurs couches.

5. Disque intervertébral artificiel selon l'une des revendications 1 à 4, dans lequel les matériaux qui constituent la bague fibreuse (4) comprennent une ou plusieurs matières plastiques, comme par exemple polyamide, polyimide, fibres de carbone ou polyétheréthercétone (PEEC), de préférence polyéthylène téréphtalate (PET).

6. Disque intervertébral artificiel selon l'une des revendications 1 à 5, dans lequel le disque (3) comprend deux parties annulaires (7, 8) agencées concentriquement et reliées l'une à l'autre de manière solidairement active.

7. Disque intervertébral artificiel selon l'une des revendications 1 à 6, dans lequel les matériaux constituant le disque (3) comprennent des élastomères de silicone ou des matériaux à base de caoutchouc, comme par exemple polycarbouréthane (PCU).

8. Disque intervertébral artificiel selon l'une des revendications 1 à 7, dans lequel les plaques terminales (1, 2) sont réalisées en un métal, de préférence en titane, en alliage de titane ou en matière plastique.

9. Disque intervertébral artificiel selon l'une des revendications 1 à 8, dans lequel les plaques terminales (1, 2) sont réalisées comme des plates-formes d'ancrage, pour atteindre une croissance des vertèbres adjacentes (W) avec les surfaces (9, 10) dirigées vers l'extérieur des plaques terminales (1, 2).

10. Disque intervertébral artificiel selon l'une des revendications 1 à 9, dans lequel l'une des plaques terminales (1, 2) comporte une gorge (11, 12) s'étendant en direction périphérique pour recevoir la bague fibreuse (4).

11. Disque intervertébral artificiel selon la revendication 10, dans lequel la bague fibreuse (4) est pressée par déformation à froid dans l'une des gorges (11, 12) pour la liaison avec l'une des plaques terminales (1, 2).

12. Disque intervertébral artificiel selon la revendication 10 ou 12, dans lequel la bague fibreuse (5) est fixée dans l'une des gorges (11, 12) par un élément de coincement (13) de forme annulaire pour la liaison avec l'une des plaques terminales (1, 2).

13. Disque intervertébral artificiel selon l'une des revendications 1 à 12, dans lequel la bague fibreuse (4) est fixée par un élément de maintien (14), de préférence au moyen d'un vissage (15) pour la liaison avec l'une des plaques terminales (1, 2).

14. Disque intervertébral artificiel selon l'une des revendications 10 à 13, dans lequel la bague fibreuse (4) est collée ou soudée dans l'une des gorges (11, 12) pour la liaison avec l'une des plaques terminales (1, 2).

15. Disque intervertébral artificiel selon l'une des revendications 10 à 14, dans lequel la bague fibreuse (4) est surmoulée de matière plastique sous forme annulaire dans l'une des gorges (11, 12) des plaques terminales (1, 2) pour la liaison avec l'une des plaques terminales (1, 2).

16. Disque intervertébral artificiel selon l'une des revendications 1 à 15, dans lequel la bague fibreuse (4) est surmoulée avec une matière plastique sur l'une des surfaces (9, 10) des plaques terminales pour la liaison avec l'une des plaques terminales (1, 2).

17. Disque intervertébral artificiel selon l'une des revendications 1 à 16, dans lequel les plaques terminales (1, 2) présentent une nervure médiane (16) en saillie, qui s'étend par exemple de la direction ventrale vers la direction dorsale.

18. Disque intervertébral artificiel selon l'une des revendications 1 à 17, dans lequel les plaques terminales (1, 2) comportent à leur bordure extérieure une denture (17) dirigée vers la vertèbre (W), de sorte que l'on obtient une liaison à coopération de formes grâce à une croissance du matériau osseux, pour la transmission de forces de torsion, de flexion et de poussée.

19. Disque intervertébral artificiel selon l'une des revendications 1 à 18, dans lequel, à l'état non chargé, la précontrainte sous laquelle le disque (3) est placé par la bague fibreuse (4) est si élevée que, sous une charge de pression maximum de par exemple 4000 N, la bague fibreuse (4) présente encore une tension résiduelle positive en direction de traction.
